# EUROPEAN PATENT SPECIFICATION

(11) **EP 2 464 448 B1**
(45) Date of publication and mention of the grant of the patent: **09.10.2013**
(21) Application number: 10744519.9
(22) Date of filing: 11.08.2010
(51) Int. Cl.: B01F 13/00, B01F 5/06, B01J 19/00, F28D 9/00

(54) **3D MICROSTRUCTURING FOR GENERATING MIXED STRUCTURES AND CHANNEL STRUCTURES IN MULTILAYER TECHNOLOGY FOR USE IN OR FOR THE CONSTRUCTION OF REACTORS**
3D-MIKROSTRUKTURIERUNG ZUR HERSTELLUNG VON MISCHSTRUKTUREN UND KANALSTRUKTUREN IN EINER MEHRSCHICHTTECHNOLOGIE ZUR VERWENDUNG FÜR REAKTOREN ODER FÜR DEN BAU VON REAKTOREN
MICROSTRUCTURATION EN 3D POUR LA PRODUCTION DE STRUCTURES MIXTES ET DE STRUCTURES DE CANAL EN TECHNOLOGIE MULTICOUCHE DESTINÉES À ÊTRE UTILISÉES DANS OU POUR LA CONSTRUCTION DE RÉACTEURS

(30) Priority: 12.08.2009 DE 102009038019
(43) Date of publication of application: 20.06.2012
(73) Proprietor: Fraunhofer-Gesellschaft zur Förderung der angewandten Forschung e.V., 80686 München (DE)
(72) Inventor: PARTSCH, Uwe, 01309 Dresden (DE); SCHNEIDER, Mareike, 01187 Dresden (DE); MÄNNEL, Dorothea, 01259 Dresden (DE); JURK, Robert, 01219 Dresden (DE); GOLDBERG, Adrian, 01259 Dresden (DE)
(74) Representative: Pfenning, Meinig & Partner GbR
(86) International application number: PCT/EP2010/004899
(87) International publication number: WO 2011/018213

(56) References cited:
- EP-A2- 1 205 670
- DE-A1- 4 235 979
- DE-U1- 20 304 101

## Description

The present invention relates to a channel structure which is configured for connection to and/or for integration into a bioreactor, a biochemical reactor, a chemical reactor and/or a reformer of an electrochemical cell (in particular of a fuel cell, of an accumulator or of a battery). The invention furthermore relates to corresponding bioreactors, biochemical reactors, chemical reactors and/or reformers having a connected and/or integrated channel structure. The invention finally relates to uses of such channel structures for supplying and removing liquids and/or gases or for mixing liquids and/or gases.

The prior art EP 1205670 A2 shows stacked channel structures which have several stacked individual layers with openings arranged in a two-dimensional regular pattern, in which the openings of adjacent layers are overlapping in a rotated and/or offset manner.

Various processes are known for producing channel structures, e.g. in the form of mixed structures: Thus, for example, stamping processes, laser ablation processes, punching processes or micro-milling processes.

However, to produce more complex channel structures formed from individual channel structures (e.g. mixed structures or evaporator structures or also defined reaction spaces), a combination of a plurality of the aforesaid processes is necessary. However, this requires a considerable effort of labor and time. In addition, there are in part problems in the individual aforesaid processes with respect to the handling capability, with respect to the manufacturability of larger aspect ratios between the channel depth and the substrate thickness, with respect to the unwanted deformation of smaller channels by mechanical effects and/or, in dependence on the materials used, with respect to unwanted vitrification or outbreaks.

Starting from the prior art, it is therefore the object of the invention to provide channel structures which can be manufactured simply, fast, flexibly, without defects and inexpensively and which can serve the supply and the mixing of media of all kinds (e.g. of reactants) in reactors or in reformers of electrochemical cells. It is furthermore an object to provide correspondingly constructed bioreactors, biochemical reactors, chemical reactors and/or reformers of electrochemical cells.

This object is achieved by a channel structure in accordance with claim 1 and by a reactor or reformer in accordance with claim 13. Uses in accordance with the invention result from claim 15. Advantageous embodiments can in each case be seen from the dependent claims.

The present invention will first be described generally in the following and then with reference to individual embodiments. The individual features of the present invention realized in combination with one another in the embodiments can in this respect also be realized in different combinations with one another within the framework given by the claims, i.e. they do not have to correspond to the example configurations shown. Individual ones of the features shown in the embodiments can also be omitted or can be combined in a different manner with further of these or other features.

The underlying idea of the present invention is to provide a plurality of individual layers which are stacked on one another and which then form the channel structure. For this purpose, the individual layers have openings (hole structures) which completely pass through the corresponding layer and which are arranged at least sectionally in the individual layers in the form of regular patterns. The layer sections of adjacent individual layers of the channel structures provided with corresponding patterns are then stacked on one another or are arranged adjacent to one another such that layer sections of directly adjacent (viewed in the layer surfaces of the individual layers) individual layers provided with such a pattern of openings overlap at least partly, wherein the openings of the overlapping layer sections are rotated and/or offset relative to one another in the respective overlap region.

A stamping process can, for example, be used to produce openings in the individual layers. The individual layers are produced so that the openings formed in them overlap only partly at least layer-section-wise, that is, are rotated and/or offset relative to one another. A variable three-dimensional channel structure which can then, for example, be configured as a mixed structure, is made possible by this partial overlapping by stacking a plurality of such individual layers on one another with openings arranged offset and/or rotated with respect to one another. Any desired shape and/or size variations of the channel structure (for example: a mixed structure) are possible by a suitable choice of the shape of the openings (for example by stamping out the openings, for example, by stamping tools with round, square or rectangular geometries), of the stringing together of individual manufacturing steps for the openings (e.g. stamping steps) and the stacking of a plurality of individual layers (in the following also: sheets) with openings. It is, however, common to respective adjacent sheets that together they have a three-dimensional guidance of channels through a partial overlap of openings (that is a rotation and/or offsetting of individual openings in the adjacent sheets relative to one another) of the different sheets.

Ceramics, in particular low-temperature cofired ceramics (so-called LTCC ceramics) or high-temperature cofired ceramics (so-called HTCC ceramics) can in particular be considered as the basis substrate for the individual layers. The individual layers can, however, also be manufactured from plastics or from mats which are suitable for stamping processes.

What is decisive for the material selection for the individual layers in this respect is that the selected material is temperature-stable, that is stable at the reaction temperatures to be selected in the reactor or in the reformer, and chemically stable, that is stable with respect to the individual reactants in the reactor or reformer.

A channel structure in accordance with the invention thus has a plurality of individual layers which are stacked on one another, wherein the individual layers each have a plurality of openings (e.g. stamped cuttings or incised holes) completely passing through the respective individual layer and wherein directly adjacent individual layers are each arranged adjacent to one another and/or are connected to one another. At least two directly adjacent individual layers each have at least one layer section whose openings are arranged in the form of a pattern respectively regular in two dimensions viewed in the respective layer surface of the respective individual layer. At least two of the layer sections of directly adjacent individual layers provided with such a pattern in this manner then overlap partially (viewed in the respective layer surfaces of these individual layers). In this respect, the openings of the two at least partly overlapping layer sections are rotated and/or offset relative to one another in the overlap region (again viewed in the respective layer surfaces of these individual layers); these openings thus only overlap in part.

Openings of directly adjacent individual layers are connected to one another by this partial overlap (that is, they form a common throughflow section for liquids and/or gases) such that channels are formed in the individual layers over a plurality of adjacent individual layers. These channels can then be connected to one another by suitable formation and arrangement of a plurality of openings in a plurality of individual layers such that a complex channel structure arises, for example in the form of a mixed structure.

A chemical reactor is understood within the framework of the present invention as a structure in which chemical processes take place and/or in which chemical conversions are carried out (this can take place, for example, at increased temperature and/or at increased pressure). A bioreactor is accordingly understood as a structure in which biological processes can be carried out. A biochemical reactor is accordingly a structure for biochemical procedures and/or for biochemical conversion. Such structures can in particular also be structures of orders of magnitude in the millimeter or micrometer ranges; the corresponding individual layers and openings of the present invention can thus have thicknesses or heights, widths and/or lengths which are formed in the range of 10 µm to 10 cm, in particular in the range from 100 µm to 1 cm.

When it is stated that a channel structure in accordance with the invention is made for integration into a reactor and/or reformer, this also includes the case that the corresponding reactor or reformer only comprises the channel structure.

In this respect, the individual layers can be formed in the manner of curved surfaces (e.g. convex or concave surfaces); as a rule, the individual layers are, however, made as planar, two-dimensional or areal layers so that as a rule the corresponding layer plane is meant when a corresponding layer surface is spoken of in the following.

A layer section is then accordingly understood as a rule as a real partial region of the corresponding layer in the layer surface (that is e.g. in the layer plane); such a layer section does not only have to comprise a part of the layer surface, but can rather also correspond to the total layer surface.

An opening is understood as a structure formed in any desired manner in the layer surface which completely passes through the corresponding individual layer in the corresponding form (that is, the material of the individual layer is removed at the location of the opening in the corresponding form). As a rule, such an opening is a hole in the individual layer having a simple structure or shape, in particular e.g. an elongate rectangular, oval or elliptical hole.

An arrangement of such openings (in the layer surface of an individual layer) in the form of a respective regular pattern in two dimensions is understood in the following as a layer section in which a plurality of openings (preferably of the same shape and of the same size) are arranged such that the openings repeat at regular intervals in two spatial directions which have an angle to one another unequal to 0° and unequal to 180°. These two spatial directions, which are preferably arranged at an angle of 90° with respect to one another, that is, stand perpendicular on one another, are also called the two dimensions of the regular pattern of openings of a layer section formed in this manner in the following.

The spacing of two directly adjacent openings in one of these two directions or dimensions is understood, provided nothing else is stated in the following, as the minimal spacing of two edge points of these openings in the corresponding direction or dimension (in the following also: edge-to-edge spacing). The spacing of the centers of mass of two openings is understood as the spacing of the geometrical centers of mass of the two openings.

When it is stated in the following that two layer sections (which are provided with a pattern of openings) of two directly adjacent individual layers overlap at least in part, this means that the two layer sections are arranged lying at least partly over one another viewed perpendicular to the layer surfaces or layer planes of these individual layers (so that the two patterns of the adjacent individual layers cover one another at least in part). The same applies accordingly to the overlap of openings: When it is stated that two openings of directly adjacent individual layers (or of the layers sections of these individual layers provided with opening patterns) overlap or cover one another in part, this means that the two openings admittedly are not arranged superimposed (in the direction perpendicular to the layer surface or layer plane) over one another, but rather have a common section or opening section viewed in the layer surface or in the layer plane so that a common aperture section or throughflow section passing through the two adjacent individual layers is formed.

When it is stated in the following that the openings of a layer section (or of a pattern) have a preferred direction, this means that they have a different extent in one direction, viewed in the layer surface, than in (at least) one other direction. In this respect, that direction is defined as the preferred direction, if nothing else is stated, in which the openings have the greatest extent (the preferred direction is then the longitudinal direction of the openings). As a rule, this preferred direction will also coincide with one of the two dimensions of the regular pattern of the openings, but this does not have to be the case. The openings can thus be arranged in a two-row and column matrix (with the two dimensions of the pattern then standing perpendicular to one another), the openings can be formed in the manner of elongate holes and the longitudinal direction of the holes can be aligned either in the column direction or in the row direction (this is then preferred direction). Alternatively to this, however, it is also possible to form the longitudinal direction of the openings in such an array pattern not along the direction of the rows or columns, but rather e.g. along a diagonal or at any desired angle (e.g. of 30°) to the direction of the columns or rows.

When it is thus stated in the following that two openings of two layer sections of directly adjacent individual layers are rotated relative to one another, this as a rule means that the preferred directions of these openings of the two patterns have an angle relative to one another of > 0° and < 180°. If two openings of two such patterns are offset relative to one another, this as a rule means that the preferred directions are admittedly arranged parallel to one another (and frequently also that the openings of the two patterns are arranged at the same level viewed in the layer surface and perpendicular to the preferred direction), but the two openings do not lie completely over one another in a superimposed manner, but rather only have, viewed in the partition surface between the two directly adjacent individual layers, a common part section in accordance with a real part of the total opening surface and/or of both opening surfaces of the two openings.

If the center of mass spacing of two directly adjacent openings of one and the same pattern or layer section of one and the same individual layer is defined in the direction of one of the two dimensions in which the pattern is made as regular as the pattern period of the corresponding pattern in this dimension, an arrangement of the openings of the patterns of two directly adjacent individual layers offset by half a period (in this dimension) means an initially identical placing over one another of the two patterns and then subsequently a displacement of one of the two patterns in the corresponding dimension direction by half this center of mass spacing.

The opening patterns of adjacent overlapping layer sections are made identical apart from a rotation and/or offset relative to one another. The openings of the individual patterns in this respect advantageously have, as described above, a preferred direction, that is, they have a larger or smaller extent in a first direction of the layer surface than in a second direction (not equal to the first direction) in the layer surface. The first direction is preferably one of the two dimensions in which the corresponding pattern is made as regular.

In a further advantageous variant, at least one of the individual layers has a plurality of layer sections with regular patterns so that the regular patterns of different layer sections do not coincide and/or have different preferred directions in at least one of the two regular pattern dimension directions.

Pairwise overlapping layer sections thus preferably result between directly adjacent individual layers, wherein the openings of the one layer section of an overlapping pair are rotated and/or offset to the openings of the other layer section of the pair.

In this respect, the openings of the two adjacent individual layers are preferably made, despite the previously described offsets and/or rotations, so that a respective opening of the one individual layer and an opening of the other individual layer form a clear connection, that is, a throughflow possibility from one opening into the other opening.

The openings of overlapping layer sections of different individual layers can be arranged rotated (with the angle of rotation preferably amounting to 90°, measured e.g. with respect to a preferred direction such as the longitudinal axial direction of the openings of one of the layer sections) and not offset relative to one another. Equally, however, it is possible to arrange the openings of overlapping layer sections of adjacent individual layers offset relative to one another and not rotated relative to one another. The offset can in this respect in particular take place by half a pattern period of one of the two dimensions of the opening pattern in one of the individual layers (or in both individual layers).

The regular pattern can in particular be made in the form of a two-dimensional matrix with rows and columns of openings (with the row direction then being perpendicular to the column direction).

The individual openings can be made in the form of elongate apertures, wherein the longitudinal axis of these elongate apertures can be aligned in the direction of one of the two aforesaid dimensions of the pattern. In this case, the longitudinal axis of these apertures stands perpendicular to the direction of the other dimension of the pattern. Viewed in the direction of the longitudinal axis, directly adjacent elongate apertures can then have an edge-to-edge distance which is smaller than the extent of such an elongate aperture in the direction of its longitudinal axis. The edge-to-edge spacing is in this respect the minimal spacing of two edge points of such elongate apertures in the direction of the longitudinal axis of the apertures (or in the direction of that dimension of the pattern in which this longitudinal axis is aligned). The edge-to-edge spacing of two directly adjacent elongate apertures in the direction of the longitudinal axis defined in this manner and the extent of an aperture in the direction of this longitudinal axis thus form the pattern period in the direction of the said dimension or in the direction of the aligned longitudinal axes. In this respect, the said edge-to-edge spacing in the longitudinal direction is preferably smaller than or equal to half, particularly preferably equal to a third of the extent of the elongate apertures in the direction of the longitudinal axis. The edge-to-edge spacing perpendicular to the direction of the above-described dimension (that is, the transverse spacing in the direction of the other of the two dimensions of the pattern)of elongate apertures arranged directly adjacent to one another can also be equal to the above-described edge-to-edge spacing in the direction of the longitudinal axis. The transverse spacing is then the minimal spacing of two edge points of adjacent apertures in the direction perpendicular to the direction of the longitudinal axis of the openings.

In a further advantageous variant, layers sections of two directly adjacent individual layers overlap so that their elongate apertures not only have the same shape and size, but are also aligned parallel to one another (that is, all face in the same direction with respect to their longitudinal axes), with an offset of the elongate apertures being realized in the direction of the longitudinal axis. The offset in this respect preferably amounts to half a pattern period in the direction of the dimension of the direction of the longitudinal axis of the pattern.

It is equally possible that two overlapping layer sections of two directly adjacent individual layers (whose elongate apertures have the same shape and size) are made with respect to their apertures that the apertures of the layer section of the one individual layer are arranged rotated relative to the apertures of the layer section of the other individual layer. The angle of rotation here preferably amounts to 90°.

A plurality of individual layers is advantageously made completely identical with respect to their layer sections, their patterns and the openings of the patterns. The individual layers made identical in this manner can then be rotated and/or offset relative to one another.

In all the variants described above, the openings of respective directly adjacent individual layers and/or layer sections are preferably made at least in part so that they overlap pairwise in real part sections. This means that two such overlapping openings of adjacent individual layers have a common intersection viewed in the layer surface or layer plane, that therefore the two openings together form a suitable passage (for the conducting of a liquid and/or of a gas, optionally also of a powder such as with a catalyst in the fixed bed or with metal hydrides as a hydrogen tank) between the two individual layers; however, without the two openings being arranged in an identical position lying over one another (the surface of the passage in the layer plane is thus smaller than the base surface of the openings in this plane).

The individual layers are preferably made in planar form and are stacked over one another in the form of a layer stack. The openings can, for example, be realized in the form of stamped cuttings or cut-outs (e.g. with the help of lasers) of part sections from the individual layers.

As will be described in more detail in the following, it is in particular possible to form a bioreactor, a biochemical reactor, a chemical reactor and/or a reformer in accordance with the invention of an electrochemical cell by stacking a plurality of individual layers in which then layer sections having regular patterns of openings are then formed in each case in a suitable manner and by suitable offset and/or rotation of the layer sections of adjacent individual layers relative to one another such that the total reactor and/or reformer comprises only these structured individual layers. (As a rule, it is still necessary for this purpose to arrange individual top or bottom layers above the topmost individual layer and below the bottommost individual layer, with as a rule either no openings or only individual openings being structured in said individual top or bottom layers, with said individual openings then serving as supply or removal channels.

The walls of the openings, that is, the inner walls of the channels of the channel structure in accordance with the invention, can in this respect be coated with a suitable catalyst which serves as a catalyst for a reaction to be carried out in the reactor.

The channel structure can furthermore also be formed for the transport of material and/or heat (e.g. as a heat exchanger).

The present invention will be described in the following with reference to a plurality of embodiments. There are shown
- Figures 1a to 1e: a first embodiment in the form of two identically structured individual layers (that is identical patterns at individual layers having openings) in the form of plastic foils which are laminated onto one another in offset form (Figure 1c) and which produce parallel channels leading in meandering form in one direction, covered with corresponding top individual layers and bottom individual layers;
- Figures 2a to 2e: a mixer structure which is formed by the introduction of an individual layer rotated by 90° between two identical and uniformly aligned individual layers (wherein then all three individual layers have one and the same pattern of openings);
- Figures 3a to 3f: mutually concentrically nested helical structures manufactured by stacking a plurality of individual layers;
- Figure 4: the sketch of a reformer for a fuel cell manufactured completely from individual layers stacked over one another and having corresponding layer sections, patterns and openings;

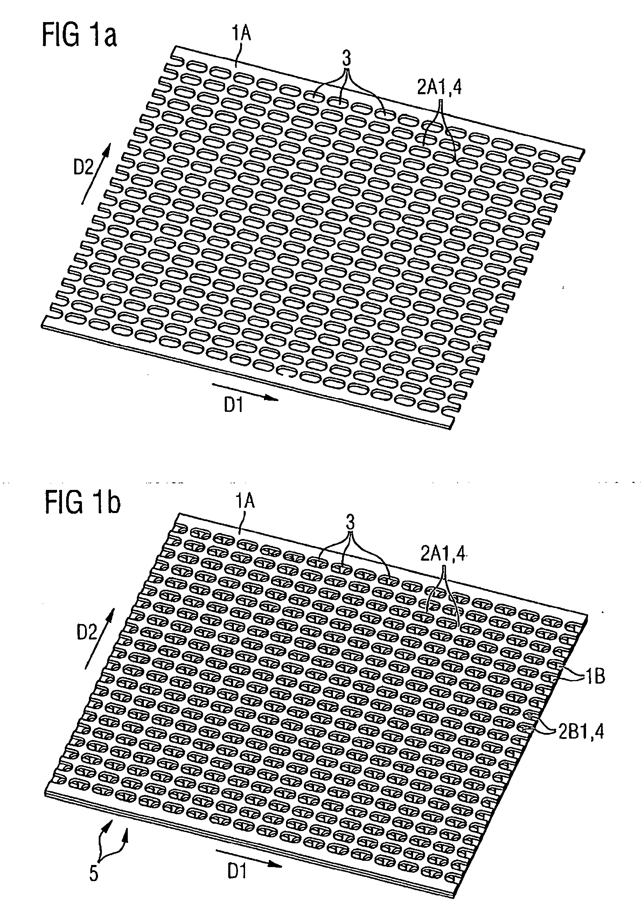

Figure 1a shows a single individual layer 1A usable for a channel structure in accordance with the invention or for stacking on one another in the form of a foil from which a plurality of openings 3 are stamped out in the form of elongate apertures. The openings 3 are arranged in the foil, which is planar here, in the form of a two-dimensional matrix, that is, in the direction of columns and rows (with the direction of the columns being perpendicular to the direction of the rows). The individual openings 3 thus form a two-dimensional perforated grid, wherein a plurality of individual holes is arranged at regular, sequential intervals in the row direction (or in the direction of a first dimension D1 of the opening pattern 4) in every row. The direction of the longitudinal axis or the longitudinal direction of the openings 3 is identical to the dimension D1 here. Equally, a plurality of openings is arranged at regular intervals behind one another in the column direction (or in the direction of the second dimension D2 of the opening pattern standing perpendicular on the first dimension D1) in every column.

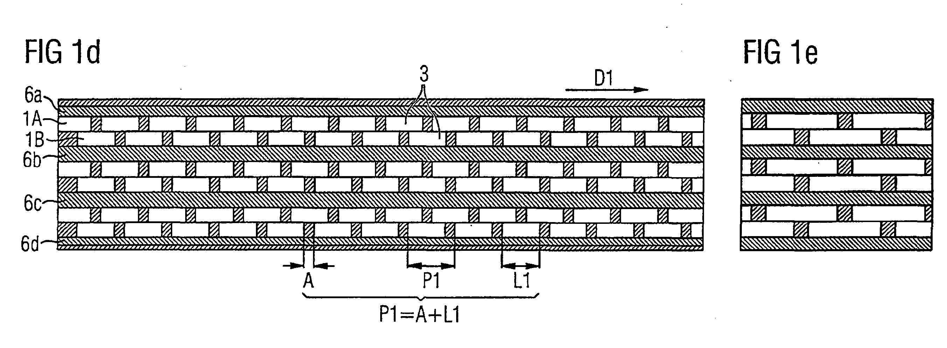

Viewed in the direction of the first dimension D1 (longitudinal direction), two directly adjacent openings or elongate apertures 3 have the edge-to-edge spacing A (cf. Figure 1d). The extent of an individual elongate aperture or of an opening 3 amounts to L1 in this longitudinal direction or direction of a first dimension D1 (cf. Figure 1d). In this respect, L1 = 3 · A in the present example. These two parameters A and L1 thus define the pattern period of the pattern 4 in the direction of the first dimension D1: This pattern period amounts to P1 = A + 1 = 4 · A In the present case, the center of mass spacing of two directly adjacent openings 3 thus equally has the value P1 in the direction of the first dimension D1. The edge-to-edge spacing perpendicular thereto or in the transverse direction here likewise amounts to A (not plotted). Since in the present case the length L1 of an opening is three times as large as the extent of the opening perpendicular thereto (transverse extent), the centre of mass spacing in the transverse direction or in the direction of the dimension D2 thus has the value 2 · A.

Figure 1a thus shows an individual layer 1A having precisely one layer section 2A1 in the form of a pattern 4 of elongate apertures 3 respectively regularly formed in the direction of the two dimensions D1, D2.

Figure 1b now shows the already described individual layer 1A and a further, identically formed second individual layer 1B lying thereunder (only one individual layer section 2B1 is thus also realized in the second individual layer 1B having a plurality of openings 3 which are in turn formed in the layer plane of the individual layer 1B in the form of a pattern 4 respectively regular in two dimensions; these two dimensions of the second individual layer 1B coincide here with the two corresponding dimensions of the first individual layer 1A due to the arrangement over one another described below in more detail of the two individual layers 1A, 1B).

The two identically formed individual layers or foils 1A, 1B are now arranged directly adjacent to one another with their layer planes parallel to one another, that is, stacked over one another in the direction perpendicular to the layer plane. This stacking is realized by an offset in the direction of the first dimension D1 (that is in the direction of the longitudinal axis of the individual openings). The two pattern matrices 4 of the upper layer 1A and of the lower layer 1B were in this respect offset by half a period P1 in the direction D1 relative to one another. No offset is realized in the direction perpendicular thereto (direction D2). Due to this one-dimensional offset of the two individual layers only in the direction of the dimension D1, a meander-like extent (viewed perpendicular to the layer plane, cf. Figure 1d) of a plurality of individual channels results, wherein these individual channels each extend in the direction of the first dimension D1. In this respect, the individual channels are, viewed in the transverse direction D2, separated from one another by the webs formed between the individual openings. The layer sections 2A1, 2B1 of the two individual layers 1A, 1B thus completely overlap one another here, that is, in the total layer plane, with the openings 3 of these two layer sections 2A1, 2B1 being offset to one another (and not rotated with respect to one another) in the overlap region 5 relative to one another by half the pattern period P1 in the direction of the first dimension D1.

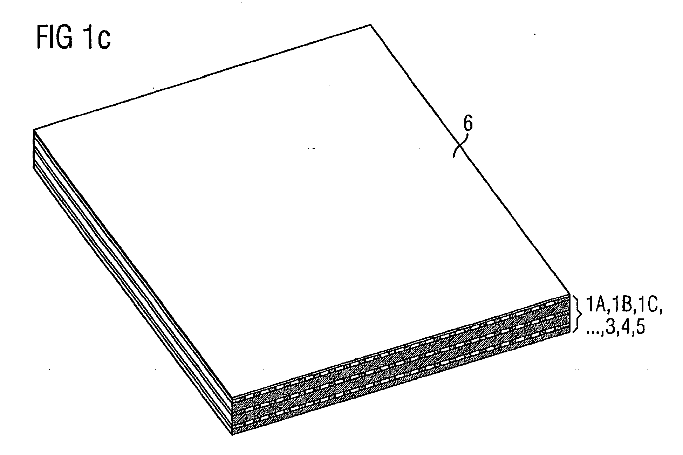

If now the upper plate 1A or its openings 3 are now covered by a closed individual layer 6a and if a plurality of such three-layered structures 6a, 1A, 1B are stacked over one another in the direction perpendicular to the layer plane, the construction shown in Figure 1d results (the further closed individual layers corresponding to the topmost sheet 6a are here provided with the reference numerals 6b, 6c, ...): A respective plurality of individual channels thus respectively extend in a plurality of planes in a meander form in the direction of the first dimensions D1. The individual layers can be laminated to one another; two foils thus result in the previously described manner having identical structures which are laminated to one another in an offset manner and are subsequently covered by a separating plane, by stacking a plurality of planes which extend over one another and in which respective parallel channels lead in a meander form in the direction of the first dimension D1. Figure 1c shows such a stack having 21 channels each in three planes in a three-dimensional plan view (the topmost individual layer corresponds to the layer 6a from Fig. 1d, but here bears the reference numeral 6).

Fig. 1e shows a sectional representation (the section is, just as in Figure 1d, perpendicular to the layer player and conducted in the direction of the first dimension D1) of a real stamped structure corresponding to the schematic representation in Figure 1d.

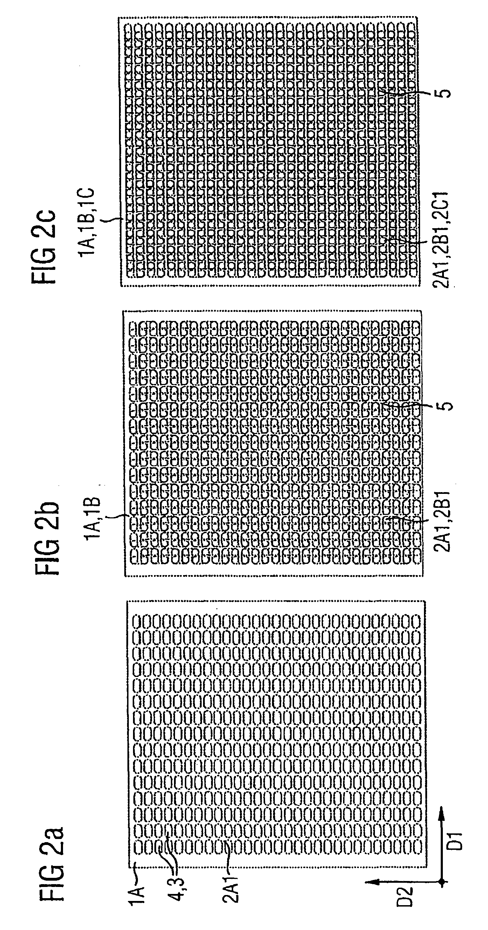

Figures 2a to 2e show a further embodiment in which the individual layers are generally formed in the same manner as is described with respect to Figures 1a to 1e (this in particular relates to the hole arrangement of the openings 3, their shapes and sizes). Only the differences will therefore be described in the following.

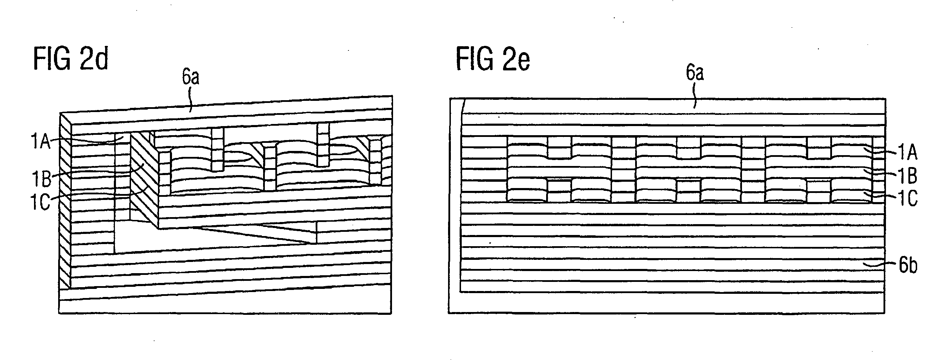

In the case shown in Figure 2, a total of three identically formed individual layers are stacked over one another perpendicular to the respective layer plane. In this respect, the bottommost layer 1A (having the individual layer section 2A1, which is formed over the total layer surface and whose pattern 4 and whose openings 3 are made as shown in Figure 1a) and the topmost third layer 1C made identical to this layer 1A are stacked over one another with an offset by half a pattern period in the direction of the first dimension D1 and of the second dimension D2 (which are equally defined as shown in Figure 1).

An intermediate layer 1B, which likewise has only one layer section 2B1 extended over the total layer plane is introduced in the manner of a sandwich between these two layers 1A and 1C and directly adjacent to these two layers. The overlap region of the three layer sections 2A1, 2B1, 2C1, as the region 5 thus corresponds to the respective total layer plane.

In contrast to the two layers 1A, 1C, however, the direction of the longitudinal axis of the openings 3 of the individual layer 1B is not aligned in the direction of the first dimension D1, but rather perpendicular thereto, that is, in the direction of the second dimension D2 (in other words, the middle individual layer 1B represents an individual layer rotated by 90° in the layer plane). The first plane 1A is thus (viewed with respect to the first dimension D1) directed lengthways, the second plane is laminated on transversely to the first plane and the third plane is in turn directed or laminated on lengthways and is offset, viewed with respect to the first plane 1A and the dimension D1, by half a pattern period.

A mixer structure thus arises by introduction of a plane 1B rotated about 90° between two planes 1A, 1C made lengthwise (with respect to their channels or openings 3). Figure 2d shows a three-dimensional section through a real, correspondingly formed mixed structure in which then the topmost individual layer is covered by a closed layer 6a (sectional representation oblique due to said mixer structure). Further individual layers having opening structures adjoin beneath the bottommost of the three individual layers having aforesaid openings. Figure 2e shows a sectional representation lengthways through the channels 3 of the mixer structure formed as described above.

Complex mixer structures can thus be constructed on a very small construction space in a simple manner with the help of the above-described individual layers and the structured formations 3, 4 of the same. In this respect, only stamping procedures are required per layer (subsequently a lamination of the individual layers must still take place) so that a fast and inexpensive structuring variant results for channel structures.

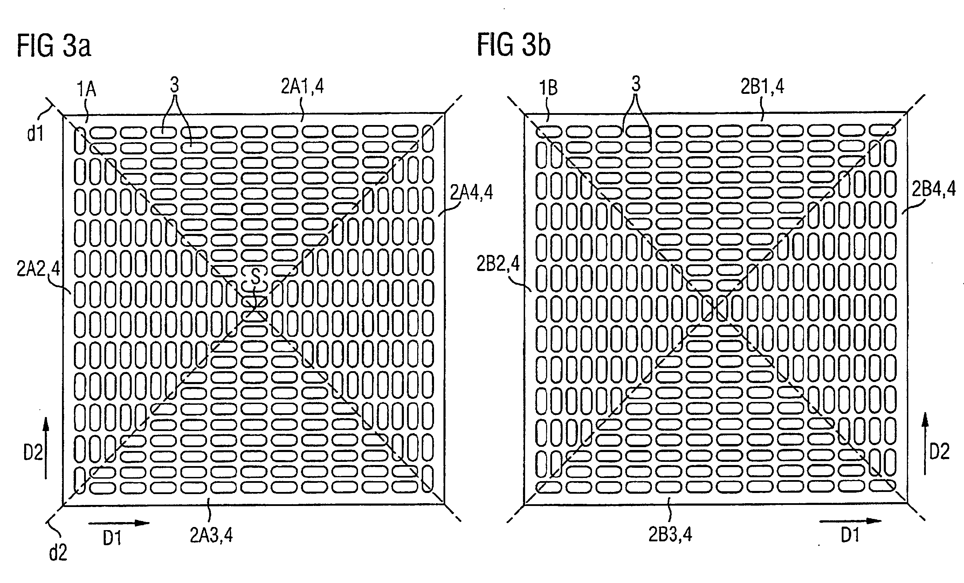

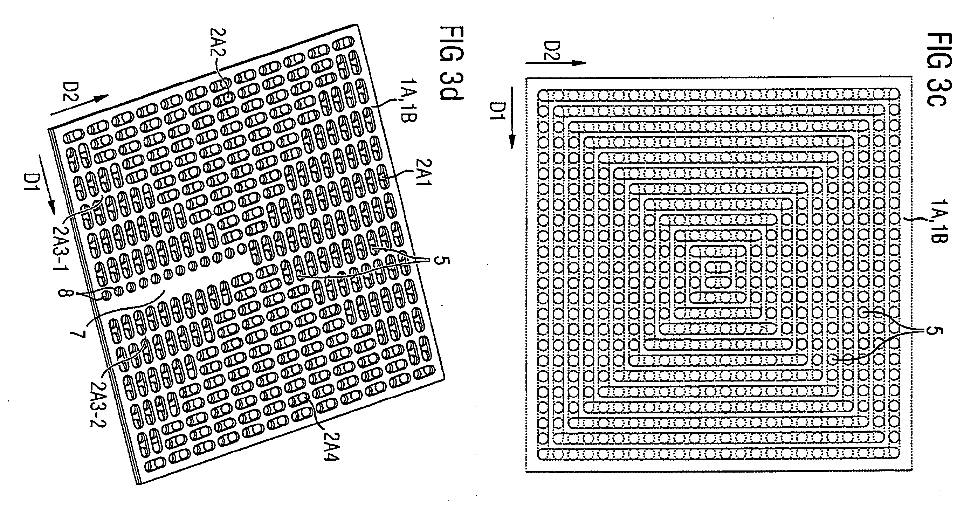

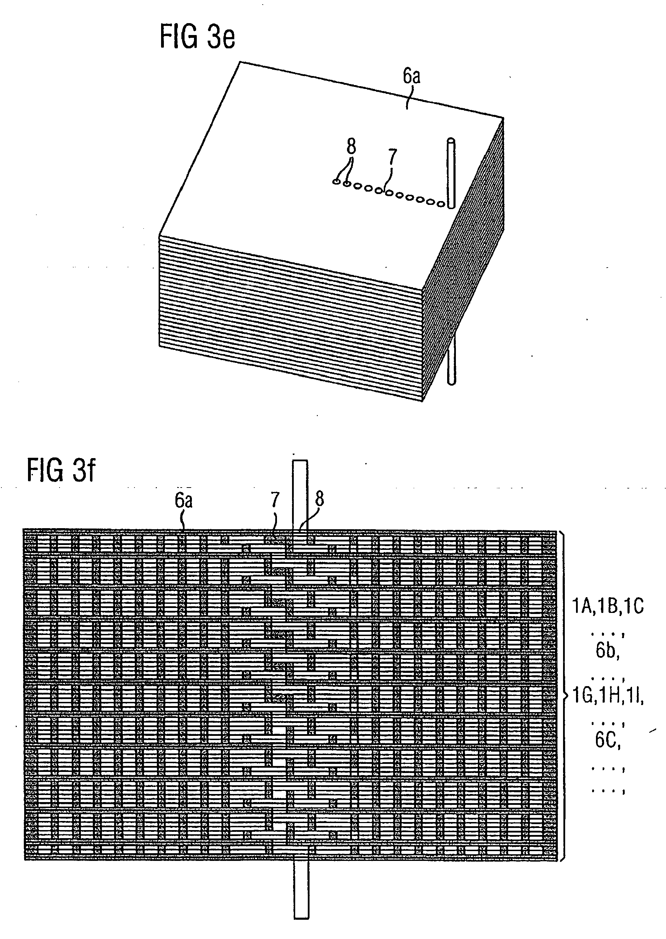

Figure 3 shows a further example for a channel structure in accordance with the invention in the form of a helical structure which can be realized within the framework of a reactor construction.

Figure 3a shows a first individual layer 1A in the form of an LTCC ceramic. The first individual layer 1A here includes a total of four individual layer sections 2A1, 2A2, 2A3 and 2A4. The four layer sections are realized in that the first individual layer 1A, square here, is divided by its two diagonals d1, d2 into four isosceles, right-angled triangles which are identical with respect to their shapes, with one of the layer sections 2A1 to 2A4 corresponding to each of these triangles.

Two respective oppositely disposed layer sections (that is the layer section pair 2A1 and 2A3 as well as accordingly also the layer section pair 2A2 and 2A4) in this respect have an identical pattern of elongate apertures 3, wherein the directions of the longitudinal axis of each aperture 3 of a layer section pair are each aligned parallel to one another. The elongate apertures 3 of the sectional section pair 2A1, 2A3 thus all extend (with their longitudinal axes) in the direction of the first dimension D1 of the four patterns 4 of the layer sections 2A1 to 2A4 and all the elongate apertures of the layer section pair 2A2, 2A4 extend (with respect to the directions of their longitudinal axes) in the direction of the second dimension D2 of the regular patterns 4 of the four layer sections 2A1 to 2A4.

In other words, all four layer sections 2A1 to 2A4 are completely filled with elongate apertures (whose shapes and sizes and whose spacings are here formed as described with respect to Figures 1 and 2), wherein the longitudinal axes of the elongate apertures are formed parallel to the triangle base of the respective layer sections. The elongate apertures of the sections 2A2 and 2A4 thus extend perpendicular to those of the layer sections 2A1 and 2A3, viewed with respect to their longitudinal axes.

Figure 3b shows a further individual section 1B which is made identical with respect to its structuring to the individual layer 1A, but which is formed in the plane shown (which corresponds to that of the layer plane) rotated by 90° about the center of mass or the center S of the individual layer 1A. If the two dimensions D1, D2 of the second individual layer 1B are thus defined identically to that of the individual layer 1A, the opening structure of the individual layer 1B is thus rotated by 90° about the center of the individual layer 1A relative to the opening structure of the individual layer 1A.

The apertures 3 of the second individual layer 1B are thus admittedly also aligned (viewed with respect to the direction of the longitudinal axes) in the two oppositely disposed layer sections 2B2 and 2B4 in the direction of the second dimension D2 and the apertures 3 of the two oppositely disposed layer sections 2B1 and 2B3 are aligned in the direction of the first dimension D1. Since, however, the outermost aperture row of the layer sections 2A2 and 2A4 (or of the layer sections 2B1 and 2B3) facing the triangle basis respectively has one more aperture 3 than the outer aperture rows of the layer sections 2A1 and 2A3 (or 2B2 and 2B4) and since thus the hole structures in the two individual layers 1A, 1B are not mirror symmetrical to the diagonals d1 and d2, a respective offset results between apertures of the two individual layers 1A, 1B lying over one another on a stacking over one another of the two individual layers 1A, 1B: The individual openings of the two layers 1A, 1B thus do not lie identically above one another, but are rather only made partly overlapping so that twelve independent annular channels result in the overlap region 5 (viewed in the layer planes) which extend concentrically into one another and have a meander-like structure viewed in the section perpendicular to the layer planes (cf. in this respect Figure 1d). Each of the twelve annular channels in this respect extends along the periphery of a square, wherein the side length of this square reduces in a linear manner from the outermost channel to the innermost channel (cf. Figure 3c).

Figure 3d now shows three individual layers stacked over one another, wherein the lower individual layer and the middle individual layer are formed in accordance with the individual layer 1A or with the individual layer 1B respectively in the Figures 3a to 3C and wherein a further individual layer (topmost layer) is arranged on this two-layer structure. Whereas the middle plate of the arrangement shown in Figure 3d (which is likewise designated by 1B here) corresponds to the individual layer 1B shown in Figure 3b, the topmost layer (which is here provided with the reference numeral 1A) has the following difference to the layer 1A of Fig. 3a (or to the respective bottommost of the three layers): Along the plumb line on the hypotenuse of the triangle of the layer section 2A3, the aperture row 3 of elongate apertures shown in Figure 3a has been replaced by a row of circular apertures 8. Since the circular apertures have a smaller extent in the direction of the first dimension D1 than the extent of the elongate apertures in the direction of their longitudinal axes, a strip-shaped, closed layer section 7 of the topmost, third individual layer 1A results parallel to this row of circular apertures, that is, likewise along the plumb line on the hypotenuse of the triangle section 2A3. The structures 7, 8 thus divide the layer section 2A3 of Fig. 3a into two smaller layers sections 2A3-1 and 2A3-2 likewise of triangular shape.

If the three-layer structure shown in Figure 3d is covered by a further, fourth individual layer (not shown) which is closed above all elongate apertures of the third individual layer 1A and only has just such circular apertures above the circular apertures 8 (wherein the circular apertures of the third layer 1A and those of the layer lying thereabove and not shown here are not made offset to one another, but are rather made lying identically over one another), a four-layer system thus results which can be stacked a multiple times over one another perpendicular to the shown layer planes (cf. Figures 3e in a three-dimensional plan view and 3f in a sectional view perpendicular to the layer planes and in the direction of the first dimension D1).

With such a stacking, a respective suitably formed, almost closed layer having a suitable channel ducting in the region of the structures 7, 8 then lies between two adjacent three-layer systems (cf. Fig. 3d) and a channel extent results (cf. Figures 3e and 3f) in the form of twelve independent individual spirals or individual helices which are nested into one another and whose helix diameter increases outwardly from the center of the total arrangement (viewed in the layer plane). The winding direction is in this respect made perpendicular to the dimensions D1, 2D.

The layers, which are made almost closed, of the total stack comprising a plurality of three-layer systems plus the almost closed layers are designated by 6a, 6b, ... in Figures 3e and 3f.

Channels separate from one another having different lengths in the layer plane are thus produced by superimposition of a plurality of identical structures or individual layers laminated in an offset manner (Figures 3a to 3d). The twelve channels shown in Figure 3c and extending concentrically about a center are thus continued perpendicular to the layer planes so that a compact helical channel ducting arises by stacking a plurality of such individual layer triples shown in Figures 3a to 3d over one another (wherein respective suitable intermediate layers are formed which are respectively closed except for the passages 7, 8 into the next pair plane, cf. Figure 3f), that is, by stacking perpendicular to the individual layer planes and by providing passages 7, 8 between the individual layers.

The above-described channel ducting of the helical structure enables an extremely compact manner of construction with a reaction surface which is simultaneously as large as possible. Each of the twelve channels extending helically is insulated hermetically per se; different reactions are thus possible in a very tight space. It is also possible to form an insulation by vacuum formation in individual channels.

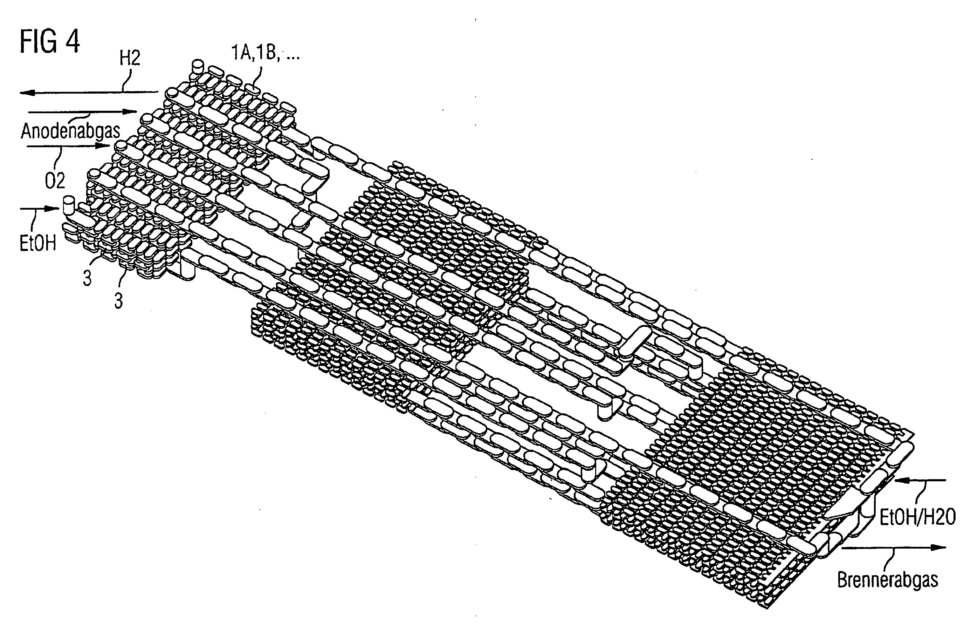

Figure 4 outlines a channel structure which is made for the integration into a reformer of a fuel cell or which forms the essential components of this reformer. The channel structure or the reformer comprises a plurality of single individual layers which are stacked over one another and into which respective layer sections are worked with their opening patterns as described above.

The reformer here comprises, viewed in the layer plane, a plurality of individual blocks which are each connected to one another by channels conducted in two layers in the manner of a meander.

As Figure 4 shows, complex channel structures can thus be realized in a manner in accordance with the invention so that the structures can represent the essential components of the reactors and/or reformers. The example reformer shown in this respect serves the reforming of ethanol.

It is in this respect in particular advantageous that any desired channel structures can be produced, with the size and shape of the channels being limited only by the stamped tools used. A very compact construction is possible with the individual layer structure shown; for the insulation of individual channels, only an individual layer thickness is necessary (thickness e.g. 200 µm). Reaction surfaces of practically any desired size can be produced for the reforming.

The present invention in particular has the following advantages:
- Channels can be made as desired in the respective degree of offset within the framework of the channel structure and can be separated from one another in a liquid-tight and/or gas-tight manner in a very small space (only one individual layer thickness is necessary for this purpose). Individual channels of the channel structure can be combined and separated from one another again as desired (mixed function). Practically any desired flow geometries can thus be produced.
- Due to the individual layer technology, very small channel webs can be formed in relation to the channel width. The channel depth in this respect depends on the number of individual layers which are laminated onto one another. Large channel surfaces or channel volumes are hereby produced in relation to the total surface or to the total volume respectively of the substrate. A high aspect ratio can thus be achieved. The channel structures, in particular the mixer structures, can be formed in the same workstep beside the usual positional marks of the screen printing and of stack markings for multilayer structures, which saves additional processing steps for the structuring. The channel structures can therefore be realized fast and with high precision.
- Conventional tools for the stamping of geometries demonstrate a low wear behavior. Stamped cuttings thereby become possible several millions of times with one tool. The prices for the replacement of such a tool are in the range of a few euros. The stamping process is thus in particular suitable as an inexpensive structuring variant for the manufacture of channel structures in accordance with the invention.
- Micro-swirls can be produced by a suitable choice of channel geometries and channel ducting or also by introducing barriers into the material guidance. They can be used specifically to improve heat transfers or material transitions.

A channel structure in accordance with the present invention may be characterized in that at least one layer section of at least one individual layer has a pattern (4) regular in two dimensions (D1, D2) having a plurality of elongate apertures as openings, with the longitudinal axis of the elongate apertures being arranged in the direction of the first (D1) of the two dimensions and with the direction of the second dimension being perpendicular to the direction of the first dimension, that is, perpendicular to this longitudinal axis.

Therein, a plurality of, preferably all the elongate apertures can be arranged, viewed in the direction of the first dimension, directly adjacent to one another and can have, viewed in this direction, an edge-to-edge spacing (A) - this spacing (A) and the extent (L1) of an elongate aperture in the direction of its longitudinal axis together define the pattern period (P1) of the first dimension (D1) - which is smaller than the extent (L1) of the elongate apertures in the direction of their respective longitudinal axes.

Therein, the edge-to-edge spacing (A) can be smaller than or equal to half, particularly preferably equal to a third of the extent (L1) of the elongate apertures in the direction of their respective longitudinal axes; and/or the edge-to-edge spacing perpendicular to the direction of the first dimension, that is, the transverse direction, of at least two, preferably all elongate apertures arranged directly adjacent to one another in the direction of the second dimension (D2), that is, perpendicular to the direction of the first dimension (D1), can be equal to the edge-to-edge spacing (A) in the direction of the first dimension.

Therein, at least two at least partly overlapping layer sections of two directly adjacent individual layers (1A, 1B, 1C) whose elongate apertures have the same shape and size, can be aligned parallel to one another and can be arranged offset relative to one another in the direction of the longitudinal axis of the elongate apertures, with the offset preferably amounting to half a pattern period (P1) of the first dimension (D1). Therein, at least two at least partly overlapping layer sections of two directly adjacent individual layers (1A, 1B, 1C) whose elongate apertures have the same shape and size and are arranged such that the elongate apertures of the layer section of the one individual layer are arranged rotated, preferably arranged rotated by 90°, relative to the elongate apertures of the layer section of the other individual layer can be provided.

A channel structure in accordance with the present invention can be characterized in that at least two directly adjacent individual layers (1A, 1B, 1C), preferably all the individual layers, are made planar so that the respective layer surface is the layer plane.

In the present invention, at least one, preferably a plurality, preferably all of the individual layers can include or can consist of a resin, a plastic, a ceramic, in particular a polyimide, a phenol resin, an epoxy resin, a silicone resin, a polyester resin, a low-temperature cofired ceramic, that is, an LTCC ceramic, and/or a high-temperature cofired ceramic, that is, an HTCC ceramic.

In the present invention, at least one, preferably a plurality of, preferably all of the openings (3) can formed by stamping out, cutting our or by laser ablation of a part section of an individual layer. The walls of the openings (3) can be coated with a catalyst at least sectionally.

## Claims

1. A channel structure which is made for connection to and/or for integration into a bioreactor, a biochemical reactor, a chemical reactor and/or a reformer of an electrochemical cell, in particular of a fuel cell, of an accumulator or of a battery, comprising
a plurality of individual layers (1A, 1B, 1C) stacked on one another, wherein the individual layers each have a plurality of openings (3) completely passing through the respective individual layer and wherein directly adjacent individual layers are each arranged adjoining one another and/or are connected to one another,
wherein at least two directly adjacent individual layers (1A, 1B, 1C) each have at least one layer section (2A1, 2A2, ..., 2B1, 2B2, ...) whose openings (3) - viewed in the respective layer surface - are arranged in the form of a pattern (4) respectively regular in two dimensions (D1, D2), and
wherein at least two of the layer sections of directly adjacent individual layers provided with such a pattern (4) overlap (5) - viewed in the layer surfaces of these individual layers - at least in part, with the openings of the two at least partly overlapping layer sections being rotated and/or offset relative to one another in the overlap region (5) - viewed in the layer surfaces of these individual layers,
**characterized in that**
the patterns (4) of the two at least partly overlapping layer sections, whose openings (3) are rotated and/or offset relative to one another, are made identical except for said rotation and/or offset.

2. A channel structure in accordance with the preceding claim,
***characterized in that***
the openings (3) of one or a plurality of layer section(s) (2A1, 2A2, ..., 2B1, 2B2, ...) of one or of a plurality of individual layer(s) each have a preferred direction, that is, have a larger or smaller extent in a first spatial direction, subsequently also referred to as longitudinal direction, in the respective layer surface than in a second spatial direction unequal to the first spatial direction in this layer surface.

3. A channel structure in accordance with the preceding claim,
***characterized in that***
the first spatial direction is the direction of the first (D1) or of the second (D2) of the two dimensions (D1, D2) of the regular pattern (4) of a layer section of an individual layer.

4. A channel structure in accordance with one of the preceding claims,
***characterized in that***
one or a plurality of individual layer(s) (1A, 1B, 1C) has/have a plurality of layer sections (2A1, 2A2, ..., 2B1, 2B2, ...) such that the openings (3) of at least two layer sections of one and the same individual layer - viewed in the layer surface - are arranged in the form of patterns (4) respectively regular in two dimensions (D1, D2) and having at least one non-coinciding dimensioning direction and/or having different preferred directions.

5. A channel structure in accordance with one of the preceding claims,
***characterized by***
at least two directly adjacent individual layers (1A, 1B, 1C) having a respective plurality of layer sections having openings (3) in the form of a pattern (4) respectively regular in two dimensions, with a plurality of layer sections of two such directly adjacent individual layers each overlapping pairwise at least in part such that the openings of the one layer section of an overlapping layer section pair are arranged rotated and/or offset to the openings of the other layer section of this overlapping layer section pair.

6. A channel structure in accordance with one of the preceding claims,
***characterized in that***
the openings of at least two at least partly overlapping layer sections of two directly adjacent individual layers (1A, 1B, 1C) are arranged at least in part in their overlap region (5) so that clear connections between openings of the two directly adjacent individual layers (1A, 1B, 1C), that is, throughflow possibilities from an opening of the one of these two directly adjacent individual layers (1A, 1B, 1C) to an opening of the other of these two directly adjacent individual layers are formed despite an offset and/or rotation of the openings relative to one another.

7. A channel structure in accordance with one of the preceding claims,
***characterized by***
at least one overlap region (5) in which the openings of the overlapping layer sections of two directly adjacent individual layers (1A, 1B, 1C) are rotated relative to one another but are not offset relative to one another.

8. A channel structure in accordance with one of the preceding claims,
***characterized by***
at least one overlap region (5) in which the openings of the overlapping layer sections of two directly adjacent individual layers (1A, 1B, 1C) are offset relative to one another but are not rotated relative to one another.

9. A channel structure in accordance with one of
the preceding claims,
***characterized by***
at least one pattern (4) regular in two dimensions (D1, D2) having a plurality of openings arranged in the form of a two-dimensional matrix in rows and in columns, with the row direction being perpendicular to the column direction.

10. A channel structure in accordance with one of the preceding claims,
***characterized in that***
at least two directly adjacent individual layers (1A, 1B, 1C) are made identical with respect to their layer sections, their patterns and their openings and - viewed in the layer surfaces of these individual layers - are arranged rotated relative to one another and/or offset relative to one another.

11. A channel structure in accordance with the preceding claim,
***characterized in that***
the two individual layers are arranged offset relative to one another, but not rotated relative to one another;
or
**in that** the two individual layers are arranged rotated relative to one another, but not offset relative to one another.

12. A channel structure in accordance with one of the preceding claims,
***characterized in that***
the openings (3) of a plurality of respectively directly adjacent individual layers (1A, 1B, 1C) and/or layer sections (2A1, 2A2, ..., 2B1, 2B2, ...) respectively only overlap in part.

13. A bioreactor, a biochemical reactor, a chemical reactor, a reformer of an electrochemical cell or an heat exchanger,
***characterized by***
at least one connected and/or integrated channel structure in accordance with one of the preceding claims; and/or in that the reactor, the reformer or the heat exchanger is made as a channel structure in accordance with one of the preceding claims.

14. A bioreactor, a biochemical reactor, a chemical reactor a reformer of an electrochemical cell or an heat exchanger in accordance with the preceding claim, wherein the total bioreactor, biochemical reactor, chemical reactor, reformer or heat exchanger is made up only of a plurality of individual layers (1A, 1B, 1C) which are stacked on one another, their layer sections (2A1, 2A2, ..., 2B1, 2B2, ...), their patterns (4) and their openings (3).

15. Use of a channel structure in accordance with one of the preceding channel structure claims for supplying one or more liquid(s), one or more gas(es) and/or one or more powder(s) to a bioreactor, a biochemical reactor, a chemical reactor or a reformer of an electrochemical cell; and/or for mixing of a plurality of liquids and/or of a plurality of gases in a bioreactor, a biochemical reactor, a chemical reactor or a reformer of an electrochemical cell; and/or for the transport of material and/or heat

## Patentansprüche

1. Kanalstruktur, die zur Verbindung mit und/oder zur Integration in einen Bioreaktor, einen biochemischen Reaktor, einen chemischen Reaktor und/oder einen Reformer einer elektrochemischen Zelle, insbesondere einer Brennstoffzelle, eines Akkus oder einer Batterie ausgelegt ist, umfassend
mehrere einzelne Schichten (1A, 1B, 1C), die aufeinander gestapelt sind, wobei die einzelnen Schichten jeweils mehrere Auslassungen (3) aufweisen, die vollständig durch die jeweilige einzelne Schicht treten, und wobei direkt benachbarte einzelne Schichten jeweils aneinander angrenzend angeordnet sind und/oder miteinander verbunden sind,
wobei mindestens zwei direkt benachbarte einzelne Schichten (1A, 1B, 1C) jeweils mindestens einen Schichtabschnitt (2A1, 2A2, ...., 2B1, 2B2,...) aufweisen, dessen Auslassungen (3) - in der jeweiligen Schichtfläche gesehen - in der Form eines Musters (4) angeordnet sind, das jeweils in zwei Abmessungen (D1, D2) regelmäßig ist, und
wobei mindestens zwei der Schichtabschnitte von direkt benachbarten einzelnen Schichten, die mit einem solchen Muster (4) versehen sind, in den Schichtflächen dieser einzelnen Schichten gesehen - zumindest teilweise überlappen (5), wobei die Auslassungen der zwei mindestens teilweise überlappenden Schichtabschnitte - in den Schichtflächen dieser einzelnen Schichten gesehen - in dem Überlappungsbereich (5) relativ zueinander gedreht und/oder versetzt sind,
**dadurch gekennzeichnet, dass**
die Muster (4) der zwei mindestens teilweise überlappenden Schichtabschnitte, deren Auslassungen (3) relativ zueinander gedreht und/oder versetzt sind, mit Ausnahme der Drehung und/oder des Versatzes identisch ausgelegt sind.

2. Kanalstruktur nach dem vorhergehenden Anspruch,
**dadurch gekennzeichnet ist, dass**
die Auslassungen (3) eines Schichtabschnitts oder mehrerer Schichtabschnitte (2A1, 2A2, ..., 2B1, 2B2, ...) einer oder mehrerer einzelner Schicht(en) jeweils eine bevorzugte Richtung aufweisen, d.h. in der jeweiligen Schichtfläche in einer ersten räumlichen Richtung, die anschließend auch als Längsrichtung bezeichnet wird, eine größere oder kleinere Erstreckung als in einer zweiten räumlichen Richtung, die ungleich der ersten räumlichen Richtung in dieser Schichtfläche ist, haben.

3. Kanalstruktur nach dem vorhergehenden Anspruch,
**dadurch gekennzeichnet, dass**
die erste räumliche Richtung die Richtung der ersten (D1) oder der zweiten (D2) der zwei Abmessungen (D1, D2) des regelmäßigen Musters (4) eines Schichtabschnitts einer einzelnen Schicht ist.

4. Kanalstruktur nach einem der vorhergehenden Ansprüche,
**dadurch gekennzeichnet, dass**
eine oder mehrere einzelne Schicht(en) (1A, 1B, 1C) mehrere Schichtabschnitte (2A1, 2A2, ..., 2B1, 2B2, ...) aufweist/aufweisen, so dass die Auslassungen (3) von mindestens zwei Schichtabschnitten von ein und derselben einzelnen Schicht - in der Schichtfläche gesehen - in der Form von Mustern (4) angeordnet sind, die jeweils in zwei Abmessungen (D1, D2) regelmäßig sind, und wobei sie mindestens eine nicht übereinstimmende Abmessungsrichtung haben und/oder unterschiedliche bevorzugte Richtungen haben.

5. Kanalstruktur nach einem der vorhergehenden Ansprüche,
**gekennzeichnet durch**
mindestens zwei direkt benachbarte einzelne Schichten (1A, 1B, 1C), die jeweils mehrere Schichtabschnitte mit Auslassungen (3) in der Form eines Musters (4) haben, das in zwei Abmessungen jeweils regelmäßig ist, wobei mehrere Schichtabschnitte von zwei solchen direkt benachbarten einzelnen Schichten sich jeweils paarweise mindestens teilweise überlappen, so dass die Auslassungen des einen Schichtabschnitts eines überlappenden Schichtabschnittpaars zu den Auslassungen des anderen Schichtabschnitts dieses überlappenden Schichtabschnittpaars gedreht und/oder versetzt angeordnet sind.

6. Kanalstruktur nach einem der vorhergehenden Ansprüche,
**dadurch gekennzeichnet, dass**
die Auslassungen von mindestens zwei teilweise überlappenden Schichtabschnitten von zwei direkt benachbarten einzelnen Schichten (1A, 1B, 1C) zumindest teilweise in ihrem Überlappungsbereich (5) so angeordnet sind, dass zwischen Auslassungen der zwei direkt benachbarten einzelnen Schichten (1A, 1B, 1C) offene Verbindungen, d.h. Durchströmmöglichkeiten, von einer Auslassung der einen dieser zwei direkt benachbarten einzelnen Schichten (1A, 1B, 1C) zu einer Auslassung der anderen dieser zwei direkt benachbarten einzelnen Schichten trotz eines Versatzes und/oder einer Drehung der Auslassungen relativ zueinander ausgebildet sind.

7. Kanalstruktur nach einem der vorhergehenden Ansprüche,
**gekennzeichnet durch**
mindestens ein Überlappungsbereich (5), in dem die Auslassungen der überlappenden Schichtabschnitte von zwei direkt benachbarten einzelnen Schichten (1A, 1B, 1C) relativ zueinander gedreht sind, aber nicht relativ zueinander versetzt sind.

8. Kanalstruktur nach einem der vorhergehenden Ansprüche,
**gekennzeichnet durch**
mindestens einen Überlappungsbereich (5), in dem die Auslassungen der überlappenden Schichtabschnitte von zwei direkt benachbarten einzelnen Schichten (1A, 1B, 1C) relativ zueinander versetzt sind, aber nicht relativ zu einander gedreht sind.

9. Kanalstruktur nach einem der vorhergehenden Ansprüche,
**gekennzeichnet durch**
mindestens ein Muster (4), das in zwei Abmessungen (D1, D2) regelmäßig ist, mit mehreren Auslassungen, die in der Form einer zweidimensionalen Matrix in Zeilen und Spalten angeordnet sind, wobei die Zeilenrichtung senkrecht zu der Spaltenrichtung ist.

10. Kanalstruktur nach einem der vorhergehenden Ansprüche,
**dadurch gekennzeichnet, dass**
mindestens zwei direkt benachbarte einzelne Schichten (1A, 1B, 1C) bezüglich ihrer Schichtabschnitte, ihrer Muster und ihrer Auslassungen identisch ausgelegt sind und - in den Schichtflächen dieser einzelnen Schichten gesehen - relativ zueinander gedreht und/oder relativ zueinander versetzt angeordnet sind.

11. Kanalstruktur nach dem vorhergehenden Anspruch,
**dadurch gekennzeichnet, dass**
die zwei einzelnen Schichten relativ zueinander versetzt, aber nicht relativ zueinander gedreht angeordnet sind;
oder
dass die zwei einzelnen Schichten relativ zueinander gedreht, aber nicht relativ zueinander versetzt angeordnet sind.

12. Kanalstruktur nach einem der vorhergehenden Ansprüche,
**dadurch gekennzeichnet, dass**
die Auslassung (3) von mehreren von jeweils direkt benachbarten einzelnen Schichten (1A, 1B, 1C) und/oder Schichtabschnitten (2A1, 2A2, ..., 2B1, 2B2, ...) sich jeweils nur teilweise überlappen.

13. Bioreaktor, biochemischer Reaktor, chemischer Reaktor, Reformer einer elektrochemischen Zelle oder Wärmetauscher,
**gekennzeichnet durch**
mindestens eine angeschlossene und/oder integrierte Kanalstruktur nach einem der vorhergehenden Ansprüche; und/oder dass der Reaktor, der Reformer oder der Wärmetauscher als Kanalstruktur nach einem der vorhergehenden Ansprüche ausgelegt ist.

14. Bioreaktor, biochemischer Reaktor, chemischer Reaktor, Reformer einer elektrochemischen Zelle oder Wärmetauscher nach dem vorhergehenden Anspruch, wobei der gesamte Bioreaktor, biochemische Reaktor, chemische Reaktor, Reformer oder Wärmetauscher nur aus mehreren einzelnen Schichten (1A, 1B, 1C), die aufeinander gestapelt sind, ihren Schichtabschnitten (2A1, 2A2, ..., 2B1, 2B2, ...), ihren Mustern (4) und ihren Auslassungen (3) bestehen.

15. Verwendung einer Kanalstruktur nach einem der vorhergehenden Ansprüche zum Zuführen einer Flüssigkeit oder mehrerer Flüssigkeiten, eines Gases oder mehrerer Gase und/oder eines Pulvers oder mehrerer Pulver zu einem Bioreaktor, einem biochemischen Reaktor, einem chemischen Reaktor oder einem Reformer einer elektrochemischen Zelle; und/oder zum Mischen von mehreren Flüssigkeiten und/oder mehreren Gasen in einem Bioreaktor, einem biochemischen Reaktor, einem chemischen Reaktor oder einem Reformer einer elektrochemischen Zelle; und/oder für den Transport von Material und/oder Wärme.

## Revendications

1. Structure de canal destinée à être raccordée à, et/ou intégrée dans un bioréacteur, un réacteur biochimique, un réacteur chimique et/ou un reformeur d'une cellule électrochimique, en particulier d'une pile à combustible, d'un accumulateur ou d'une batterie, comprenant
une pluralité de couches individuelles (1A, 1B, 1C) empilées les unes sur les autres, dans laquelle les couches individuelles présentent chacune une pluralité d'ouvertures (3) qui passent complètement à travers la couche individuelle respective et dans laquelle les couches individuelles directement adjacentes sont agencées chacune contiguës les unes aux autres et/ou sont raccordées les unes aux autres ;
dans laquelle au moins deux couches individuelles directement adjacentes (1A, 1B, 1C) présentent chacune au moins une section de couche (2A1, 2A2,..., 2B1, 2B2, ...) dont les ouvertures (3), vues dans la surface de couche respective, sont agencées sous la forme d'un motif (4) respectivement régulier dans deux dimensions (D1, D2) ; et
dans laquelle au moins deux des sections de couche de couches individuelles directement adjacentes dotées d'un tel motif (4) se chevauchent (5), vues dans les surfaces de couche de ces couches individuelles, au moins en partie, les ouvertures des deux sections de couche qui se chevauchent au moins partiellement étant tournées et/ou décalées les unes par rapport aux autres dans la région de chevauchement (5), vues dans les surfaces de couche de ces couches individuelles ;
**caractérisée en ce que**
les motifs (4) des deux sections de couche qui se chevauchent au moins partiellement, dont les ouvertures (3) sont tournées et/ou décalées les unes par rapport aux autres, sont identiques sauf pour ladite rotation et/ou pour ledit décalage.

2. Structure de canal selon la revendication précédente,
**caractérisée en ce que**
les ouvertures (3) d'une ou d'une pluralité de sections de couche (2A1, 2A2, ..., 2B1, 2B2, ...) d'une ou d'une pluralité de couches individuelles présentent chacune une direction préférée, c'est-à-dire, présentent une étendue plus grande ou plus petite dans une première direction spatiale, désignée également par la suite sous le nom de direction longitudinale, dans la surface de couche respective, que dans une seconde direction spatiale différente de la première direction spatiale dans cette surface de couche.

3. Structure de canal selon la revendication précédente,
**caractérisée en ce que**
la première direction spatiale est la direction de la première (D1) ou de la seconde (D2) des deux dimensions (D1, D2) du motif régulier (4) d'une section de couche d'une couche individuelle.

4. Structure de canal selon l'une quelconque des revendications précédentes,
**caractérisée en ce que**
une ou une pluralité de couches individuelles (1A, 1B, 1C) présentent une pluralité de sections de couche (2A1, 2A2, ..., 2B1, 2B2, ...) de sorte que les ouvertures (3) d'au moins deux sections de couche d'une et même couche individuelle, vues dans la surface de couche, sont agencées sous forme de motifs (4) respectivement réguliers dans deux dimensions (D1, D2) et présentant au moins une direction de dimensionnement non coïncidente et/ou présentant des directions préférées différentes.

5. Structure de canal selon l'une des revendications précédentes,
**caractérisée par**
au moins deux couches individuelles directement adjacentes (1A, 1B, 1C) présentant une pluralité respective de sections de couche présentant des ouvertures (3) sous la forme de motifs (4) respectivement réguliers dans deux dimensions, avec une pluralité de sections de couche de deux couches individuelles directement adjacentes de ce type se chevauchant chacune par paires au moins en partie de sorte que les ouvertures de l'une des sections de couche d'une paire de sections de couche se chevauchant, sont agencées en étant tournées et/ou décalées par rapport aux ouvertures de l'autre section de couche de cette paire de sections de couche se chevauchant.

6. Structure de canal selon l'une quelconque des revendications précédentes,
**caractérisée en ce que**
les ouvertures d'au moins deux sections de couche se chevauchant au moins partiellement de deux couches individuelles directement adjacentes (1A, 1B, 1 C) sont agencées au moins en partie dans leur région de chevauchement (5) de sorte que des raccordements nets entre les ouvertures des deux couches individuelles directement adjacentes (1A, 1B, 1C), c'est-à-dire, des possibilités d'acheminement à partir d'une ouverture de l'une de ces deux couches individuelles directement adjacentes (1A, 1B, 1C) vers une ouverture de l'autre de ces deux couches individuelles directement adjacentes, sont formées malgré un décalage et/ou une rotation des ouvertures l'une par rapport à l'autre.

7. Structure de canal selon l'une quelconque des revendications précédentes,
**caractérisée par**
au moins une région de chevauchement (5) dans laquelle les ouvertures des sections de couche se chevauchant de deux couches individuelles directement adjacentes (1A, 1B, 1C) sont tournées l'une par rapport à l'autre mais ne sont pas décalées l'une par rapport à l'autre.

8. Structure de canal selon l'une quelconque des revendications précédentes,
**caractérisée par**
au moins une région de chevauchement (5) dans laquelle les ouvertures des sections de couche se chevauchant de deux couches individuelles directement adjacentes (1A, 1B, 1C) sont décalées l'une par rapport à l'autre mais ne sont pas tournées l'une par rapport à l'autre.

9. Structure de canal selon l'une des revendications précédentes,
**caractérisée par**
au moins un motif (4) régulier dans deux dimensions (D1, D2) présentant une pluralité d'ouvertures agencées sous la forme d'une matrice bidimensionnelle en lignes et en colonnes, avec la direction des lignes étant perpendiculaire à la direction des colonnes.

10. Structure de canal selon l'une quelconque des revendications précédentes,
**caractérisée en ce que**
au moins deux couches individuelles directement adjacentes (1A, 1B, 1C) sont identiques en ce qui concerne leurs sections de couche, leurs motifs et leurs ouvertures et, vues dans les surfaces de couche de ces couches individuelles, sont agencées en étant tournées l'une par rapport à l'autre et/ou décalées l'une par rapport à l'autre.

11. Structure de canal selon la revendication précédente,
**caractérisée en ce que**
les deux couches individuelles sont agencées en étant décalées l'une par rapport à l'autre, mais ne sont pas tournées l'une par rapport à l'autre ;
ou
**en ce que** les deux couches individuelles sont agencées en étant tournées l'une par rapport à l'autre, mais ne sont pas décalées l'une par rapport à l'autre.

12. Structure de canal selon l'une quelconque des revendications précédentes,
**caractérisée en ce que** :
les ouvertures (3) d'une pluralité, respectivement, de couches individuelles directement adjacentes (1A, 1B, 1C) et/ou de sections de couche (2A1, 2A2,..., 2B1, 2B2, ...) se chevauchent respectivement seulement en partie.

13. Bioréacteur, réacteur biochimique, réacteur chimique, reformeur d'une cellule électrochimique ou échangeur de chaleur,
**caractérisé par**
au moins une structure de canal raccordée et/ou intégrée selon l'une des revendications précédentes ; et/ou
en ce que le réacteur, le reformeur ou l'échangeur de chaleur est fabriqué sous forme d'une structure de canal selon l'une des revendications précédentes.

14. Bioréacteur, réacteur biochimique, réacteur chimique, reformeur d'une cellule électrochimique ou échangeur de chaleur selon la revendication précédente, dans lequel la totalité du bioréacteur, du réacteur biochimique, du réacteur chimique, du reformeur ou de l'échangeur de chaleur est constitué, uniquement, d'une pluralité de couches individuelles (1A, 1B, 1C), qui sont empilées les unes sur les autres, de leurs sections de couche (2A1, 2A2, ..., 2B1, 2B2, ...), de leurs motifs (4) et de leurs ouvertures (3).

15. Utilisation d'une structure de canal selon l'une quelconque des revendications de structure de canal précédentes, destinée à
fournir un ou plusieurs liquides, un ou plusieurs gaz et/ou une ou plusieurs poudres à un bioréacteur, un réacteur biochimique, un réacteur chimique ou un reformeur d'une cellule électrochimique ; et/ou
mélanger une pluralité de liquides et/ou une pluralité de gaz dans un bioréacteur, un réacteur biochimique, un réacteur chimique ou un reformeur d'une cellule électrochimique ; et/ou
acheminer un matériau et / ou de la chaleur.
